# EUROPEAN PATENT SPECIFICATION

(11) **EP 3 283 179 B1**
(45) Date of publication and mention of the grant of the patent: **27.01.2021**
(21) Application number: 16708359.1
(22) Date of filing: 08.03.2016
(51) Int. Cl.: A61K 8/02, A61K 8/06, A61K 8/26, A61K 8/28, A61Q 19/00, A61K 8/92, A61K 8/34, A61K 8/36, A61K 8/31, A61K 8/39, A61K 8/86

(54) **SOLID SKIN CARE EMULSION COMPRISING SOLID AND LIQUID LIPIDS**
FESTE HAUTPFLEGEEMULSION ENTHALTEND FESTE UND FLÜSSIGE LIPIDE
EMULSION SOLIDE Ä SOIN DE LA PEAU CONTENANT LES LIPIDES SOLIDES ET LIQUIDES

(30) Priority: 31.03.2015 US 201562140684 P
(43) Date of publication of application: 21.02.2018
(73) Proprietor: Beiersdorf AG, 20253 Hamburg (DE)
(72) Inventor: HALLMANN, Marita, 22417 Hamburg (DE); ESPADA VARGAS, Karina, 37270 Léon (MX); KÖHLER, Manuela, 22527 Hamburg (DE); STREICHER, Harald, 22395 Hamburg (DE)
(86) International application number: PCT/EP2016/000403
(87) International publication number: WO 2016/155867

(56) References cited:
- WO-A1-2015/028424
- CN-A- 104 435 240
- GB-A- 609 698

## Description

### BACKGROUND OF THE INVENTION

### 1. Field of the Invention

The present invention relates to a solid cosmetic skin care emulsion and specifically, to a cosmetic composition (preferably a shower composition) for skin care which is intended and suitable for use on already cleansed (wet or moist) skin, although it also provides skin care benefits also when applied onto dry skin. The present invention also relates to a method of skin care while showering and to a method of solidifying a skin care emulsion.

### 2. Discussion of Background Information

Cosmetic or dermatological preparations can be divided on the basis of their application time and their application purpose. Some products are immediately washed off after application ("rinse-off"), others are intended to remain on the skin for longer periods of time and are effective there ("leave-on").

Cosmetic preparations for skin care are primarily developed for application to dry skin. This form of preparations is known as leave-on preparations, such as creams, lotions or body milk. Often, these are formulated as emulsions, in particular W/O, O/W, O/W/O or W/O/W emulsions or hydrogels.

Emulsions are generally understood as meaning heterogeneous systems which comprise two liquids that are immiscible or only miscible to a limited extent and which are usually referred to as phases. In an emulsion, one of the two liquids (water or oil) is dispersed in the form of very fine droplets in the other liquid. The liquids (pure or in the form of solutions) are present in an emulsion in a more or less fine distribution, which is generally only stable to a limited extent.

If the two liquids are water and oil and oil droplets are present in fine distribution in water, then this is an oil-in-water emulsion (O/W emulsion, e.g. milk). The basic character, for example electrical conductivity, sensory properties, ability of the continuous phase to stain, of an O/W emulsion is defined by the water. In the case of a water-in-oil emulsion (W/O emulsion, e.g. butter), the principle is reversed, the basic character here being determined by the oil.

Leave-on preparations are not suitable for application to wet or moist skin. Due to the emulsifiers present, they are able to emulsify water and, due to the lipids, in some cases leave behind an oily film.

Rinse-off preparations are designed for application under the shower or during bathing. By contrast, rinse-off preparations, however, involve to a lesser extent the care aspect as is obtained upon rubbing cream in.

It is desirable to provide a preparation which exhibits a skin care effect and can be applied not only on dry skin but also as rinse-off preparation, for example under the shower.

It also is desirable to provide a corresponding preparation in solid form, e.g., in the form of a solid piece such as, e.g., a bar, instead of a more or less viscous liquid or gel.

EP 1 390 006 A2 discloses oil-in-water emulsions for application to wet skin. The preparations of EP 1 390 006 A2 comprise water, a dispersion stabilizer, a structured oil phase and structurant, which form a stable network of distantly distributed solids in the liquids. Besides preferred inorganic structurants, solid fatty acid esters and vaseline are specified *inter alia* as organic structurants.

EP 2 174 639 A2 discloses oil-in-water emulsions for application to wet skin. The preparations comprise water-soluble polymer, pasty oils and liquid oil in combination with a large amount of glycerol. Examples of water-soluble polymers are *inter alia* natural polymers such as vegetable polysaccharides, animal proteins, semi-synthetic polymers such as cellulose, starch, alginates, polysaccharide derivatives, synthetic polymers such as vinyl polymers such as polyvinyl alcohol, polyvinylpyrrolidone, polyvinyl methyl ether, carboxyvinyl polymers, alkyl-modified carboxyvinyl polymers (acrylate-alkyl methacrylate copolymer, etc.) and sodium polyacrylate, and also polyethylene glycol and ethylene oxide-propylene oxide block copolymers.

WO 2013/064391 A2 discloses an aqueous cosmetic or dermatological preparation for application on wet or moist skin which is substantially emulsifier-free and comprises at least two different polyacrylic acid polymers, at least two different C14-22 fatty alcohols, and at least about 13% by weight of microcrystalline wax, preferably in combination with one or more hydrocarbon oils.

GB 2 361 641 A discloses a cosmetic lotion in the form of a bar which comprises from 16 % to 76 % by weight of cocoa butter. The lotion contains water-based and oil-based ingredients and the cocoa butter is stated to play the role of an emulsifier and to be responsible for the solid consistency of the lotion.

Additionally, several shower bar products for skin care a currently on the market such as, e.g., the King of Skin, Buffy, Schnuggle and Aqua Mirabilis in-shower bars available from the company Lush, the Solid Bath Oil in-shower bar available from the company Heymountain Cosmetics, and the Body Butter Bar Silky in-shower bar available from the company Manor Hall Soap Company, to name just a few. These commercial products have in common that they do not contain water.

### SUMMARY OF THE INVENTION

The present invention provides a cosmetic skin care composition which is suitable for application onto dry skin or and in particular, onto (already cleansed) wet or moist skin. The composition is present as an emulsion which is solid up to a temperature of at least 35° C and comprises (i) one or more solid lipids, (ii) one or more liquid lipids, (iii) one or more non-ionic emulsifiers and (iv) one or more (preferably solid) inorganic substances which are capable of solidifying the emulsion (preferably by binding water), selected from aluminum and/or zirconium compounds.

In one aspect, the composition of the present invention may be solid up to temperature of at least 40° C, and particularly up to a temperature of at least 45° C.

In another aspect, the composition may be an oil-in-water emulsion.

In yet another aspect, the one or more solid lipids which are present in the composition of the present invention may comprise a hydrocarbon wax, preferably at least microcrystalline wax, and/or an ester wax (e.g., derived from plant or animal sources), preferably at least hydrogenated castor oil.

In a still further aspect of the composition of the present invention, the one or more liquid lipids may comprise hydrocarbon oil, preferably at least liquid paraffin (paraffinum liquidum) or a similar hydrocarbon oil.

In another aspect of the composition of the present invention, the one or more non-ionic emulsifiers contained therein may comprise one or more saturated or unsaturated fatty acids (preferably selected from C₁₄₋₂₂ fatty acids such as, e.g., palmitic acid, stearic acid, myristic acid, arachidic acid, and oleic acid) and/or one or more saturated or unsaturated fatty alcohols (preferably at least cetyl alcohol and/or stearyl alcohol) and/or one or more ethoxylated saturated or unsaturated fatty alcohols and/or one or more saturated or unsaturated propoxylated fatty alcohols and/or one or more ethoxylated and propoxylated saturated or unsaturated fatty alcohols (preferably one or more ethoxylated and/or propoxylated oleyl alcohols and/or one or more ethoxylated and/or propoxylated stearyl alcohols such as, e.g., oleth-20 and PPG-15 stearyl ether).

In another preferred aspect of the composition of the present invention, the one or more inorganic substances which are capable of solidifying the emulsion selected from aluminum chloride, aluminum chlorohydrate, aluminum sesquichlorohydrate, aluminum zirconium trichlorohydrate glycine, aluminum zirconium tetrachlorohydrate glycine, aluminum zirconium pentachlorohydrate glycine, potassium aluminum sulfate.

In yet another aspect, the composition of the present invention may comprise, based on the total weight of the composition, at least 30 %, e.g., at least 35 %, or at least 40 % by weight of water and/or from 15 % to 40 % , e.g., from 20 % to 30 % by weight of component (i) and/or from 5 % to 20 %, e.g., from 7 to 15 % by weight of component (ii) and/or from 10 % to 30 %, e.g., from 15 % to 25 % by weight of component (iii) and/or from 0.5 % to 25 % by weight, e.g., from 1 % to 20 % by weight, or from 2 to 15 % by weight of component (iv).

In another aspect, the composition may further comprise talc, preferably in a concentration of up to 15 %, e.g., up to 10 % by weight, based on the total weight of the composition.

The present invention also provides a cosmetic skin care (preferably shower) composition for use on already cleansed (wet or moist) skin, which composition is an oil-in-water emulsion that is solid up to a temperature of at least 40° C and comprises, based on the total weight of the composition, from 18 % to 25 % by weight of (i) one or more solid lipids, from 7 % to 15 % by weight of (ii) one or more liquid lipids, from 15 % to 25 % by weight of (iii) one or more non-ionic emulsifiers, from 1% to 15% by weight, preferably from 2 % to 5 % by weight of (iv) one or more inorganic aluminum and/or zirconium compounds which are capable of binding water to thereby solidify the emulsion, and at least 35 % by weight of water.

In one aspect of the composition, component (i) may comprise microcrystalline wax and hydrogenated castor oil and/or component (ii) may comprise liquid paraffin and/or component (iii) may comprise one or more C₁₄₋₂₀ fatty acids, one or more C₁₄₋₁₈ fatty alcohols (preferably at least cetyl alcohol and stearyl alcohol), one or more ethoxylated C₁₄₋₁₈ fatty alcohols (preferably at least oleth-20), and one or more propoxylated C₁₄₋₁₈ fatty alcohols (preferably at least PPG-15 stearyl ether).

In another aspect, the composition may comprise as component (iv) one or both of aluminum chloride and aluminum chlorohydrate.

In yet another aspect, the composition may further comprise talc, preferably in a concentration of up to 10 % by weight.

In another aspect of the compositions of the present invention as set forth above, the compositions may be present in the form of a solid piece, preferably a bar.

In another aspect, the compositions may be free of surfactants.

The present invention also provides a method of skin care during showering. The method comprises cleansing the skin, contacting the cleansed skin with a composition according to the present invention as set forth above (including the various aspects thereof) to apply the composition to skin, and thereafter rinsing the skin with water.

The present invention also provides a method of solidifying a skin care emulsion which comprises one or more solid lipids, one or more liquid lipids, and one or more non-ionic emulsifiers. The method comprises incorporating in the emulsion one or more inorganic substances which are capable of solidifying the emulsion (preferably by binding water) in an amount that is sufficient to solidify the emulsion, wherein the one or more inorganic substances comprise aluminum and/or zirconium compounds.

### DETAILED DESCRIPTION OF THE PRESENT INVENTION

The particulars shown herein are by way of example and for purposes of illustrative discussion of the embodiments of the present invention only and are presented in the cause of providing what is believed to be the most useful and readily understood description of the principles and conceptual aspects of the present invention. In this regard, no attempt is made to show structural details of the present invention in more detail than is necessary for the fundamental understanding of the present invention, the description making apparent to those skilled in the art how the several forms of the present invention may be embodied in practice.

As used herein, the singular forms "a," "an," and "the" include the plural reference unless the context clearly dictates otherwise. For example, reference to "a compound" would also mean that mixtures of two or more compounds can be present unless specifically excluded.

Except where otherwise indicated, all numbers expressing quantities of ingredients, reaction conditions, etc. used in the instant specification and appended claims are to be understood as being modified in all instances by the term "about." Therefore, each numerical parameter should be construed in light of the number of significant digits and ordinary rounding conventions.

As set forth above, the cosmetic shower composition according to the present invention is present as an emulsion which is solid up to a temperature of at least 35° C. While the emulsion can be any type of emulsion such as, e.g., a W/O, O/W, O/W/O or W/O/W emulsion or a microemulsion, it is preferred for the emulsion to be an O/W emulsion. It further is preferred for the emulsion to be solid up to a temperature of at least 40° C and in particular, up to a temperature of at least 45° C. Preferably, the composition is in the form of a solid piece such as a bar, particularly a bar of the size and shape of conventional soap bars (and/or commercially available in-shower bars).

The one or more solid lipids which are present in the composition of the present invention will usually comprise or consist of waxes and wax-like substances, and preferably comprise or essentially consist of lipids which are selected from hydrocarbon waxes and waxes of plant or animal origin. Within the context of the present disclosure, the expression "lipids" is used as a generic term for fats, oils, waxes and the like, as is well known to those of skill in the art.

A preferred example of a solid lipid for use in the composition of the present invention is microcrystalline wax. Microcrystalline wax is a generic term and alternative names therefor include Cera Microcristallina. Microcrystalline wax is a type of wax produced by de-oiling petrolatum, as part of the petroleum refining process. In contrast to the more familiar paraffin wax which contains mostly unbranched alkanes, microcrystalline wax contains a higher percentage of isoparaffinic (branched) hydrocarbons and naphthenic hydrocarbons. It exhibits finer crystals than paraffin wax. It predominantly consists of high molecular weight saturated aliphatic hydrocarbons having more than 35 carbon atoms in the molecule. It is generally darker, more viscous, denser, tackier and more elastic than paraffin wax, and has a higher molecular weight and melting point. The elastic and adhesive characteristics of microcrystalline wax are related to the non-straight chain components which it contains. Typical microcrystalline wax crystal structure is small and thin, making it more flexible than paraffin wax. A microcrystalline wax which is suitable for use in the present invention has the CAS No. 63231-60-7 (and or EINECS/EILINCS No. 264-038-1).

Microcrystalline wax when produced by wax refiners is typically produced to meet a number of ASTM specifications such as congeal point, needle penetration, color, and viscosity. Microcrystalline wax can generally be categorized into "laminating" grades and "hardening" grades. The laminating grades typically have a melting point of 140-175° F and a needle penetration of 25 or above. The melting point of hardening grades will usually range from about 175-200° F, and the needle penetration thereof will usually be 25 or below. Both grades are suitable for use in the present invention.

Microcrystalline wax is derived from the refining of the heavy distillates from lubricant oil production. This by-product then must be de-oiled at a wax refinery. Depending on the end use and desired specification, the product then may have its odor removed and color removed. This is usually done by means of a filtration method or by hydro-treating the wax material.

Microcrystalline wax for use in the instant invention will usually be subject to high quality standards. The microcrystalline wax for use in the instant invention usually will be substantially free from, for example, polycyclic aromatics, sulfur-containing compounds and other allergens such as, e.g., crop protection agents. Due to its chemical neutrality microcrystalline wax has no allergenic potential. Allergenic reactions triggered by microcrystalline wax are hitherto unknown. Compared to animal or plant oils, microcrystalline wax has a high oxidation stability, i.e., does not become rancid and requires no additional stabilizers. Microcrystalline wax and thus, also the preparations containing it therefore also require no or relatively small amounts of additional preservatives.

The skin care properties of microcrystalline wax are primarily in the area of skin moisturization. Microcrystalline wax forms a partially occlusive protective film on the skin which protects it against drying out. This is very important particularly in the case of dry skin or highly stressed skin with a damaged skin barrier. Partially occlusive care products position themselves in the upper horny layer and thereby reduce the transepidermal water loss. In combination with skin moisturizers (e.g., glycerol), they help to rapidly restore the equilibrium of the skin. It is noted that very similar substance mixtures, the so-called mineral waxes, are naturally present in relatively large amounts also in various plant waxes (e.g. candelilla wax) and insect waxes (e.g. beeswax). These waxes are also suitable for use in the present invention.

If present, microcrystalline wax will usually be present in the composition of the present invention in a concentration of preferably at least 8 % by weight, e.g., at least 10 % by weight, at least 11 % by weight, at least 12 % by weight, or at least 13 % by weight, and will usually not be present in a concentration of higher than 20 % by weight, e.g., higher than 18 % by weight, higher than 17 % by weight, higher than 16 % by weight, or higher than 15 % by weight.

The solid lipids of the composition of the present invention preferably comprise also one or more lipids of plant or animal origin such as esters of glycerol with (optionally modified) fatty acids. A preferred example of corresponding lipid is castor wax, also called hydrogenated castor oil. Hydrogenated castor oil is a hard, brittle, vegetable wax. It is usually produced by the hydrogenation of pure castor oil (obtained from castor beans). It is odorless and insoluble in water.

If present, hydrogenated castor oil will be present in the composition of the present invention in a concentration of preferably at least 3 % by weight, e.g., at least 4 % by weight, at least 5 % by weight, or at least 6 % by weight, and will usually not be present in a concentration of higher than 15 % by weight, e.g., not higher than 10 % by weight, not higher than 8 % by weight, or not higher than 7 % by weight.

The one or more liquid lipids which are present in the composition of the present invention preferably are or essentially consist of hydrocarbon oils. A preferred hydrocarbon oil for use in the instant invention is medical white oil, also called paraffinum liquidum. Medical white oils are substance mixtures which have a varying composition depending on origin. For example, products which have been obtained from geologically old Venezuelan petroleum are particularly rich in naphthenes (cycloalkanes). By contrast, the geologically young North Sea oil is low in naphthenes and comprises predominantly acyclic compounds.

Naphthene-rich mineral oils only occur in selected areas of the world (Venezuela, Saudi Arabia, Russia). They are difficult to obtain and accordingly expensive. Low-naphthene mineral oils are easier to obtain and are rather to be classed as good value. A disadvantage of the low-naphthene mineral oils is that these oils or mixtures with these oils with, *inter alia,* microcrystalline wax used in emulsions destabilize the emulsions, which results in a severe oil separation.

Naphthenes or alicyclic hydrocarbons are ring-shaped hydrocarbons. The naphthene content of crude oil is generally 5 %, in the case of Russian oil it is often more than this, and in the case of American oil below this. Naphthenes have a higher bond tension than paraffins in the molecular structure and therefore have a higher heating value.

Cycloalkanes (cycloparaffins) are saturated ring-shaped hydrocarbons of the general formula CₙH₂ₙ (n = 3, 4, 5 ...), the names of which are formed from that of the corresponding alkane and the prefix cyclo-. The cycloalkanes, *inter alia,* cyclopentane and cyclohexane, occurring in petroleum are also called naphthenes.

Preferably, naphthene-containing medical white oil is used in the composition of the instant invention.

Paraffinum liquidum and/or any other hydrocarbon oil will usually be present in the preparation of the present invention in a concentration of at least 2 % by weight, e.g., at least about 3 % by weight, at least about 4 % by weight, or at least about 5 % by weight, but usually not higher than 10 % by weight, e.g., not higher than 9 % by weight, not higher than 8 % by weight not, or not higher than 7 % by weight. Further, the total concentration of microcrystalline wax and paraffinum liquidum will often be at least 8 % by weight, e.g., at least 10 % by weight, at least 12 % by weight, or at least 14 % by weight, but will usually be not higher than 25 % by weight, e.g., not higher than about 20 % by weight, not higher than 18 % by weight, or not higher than 16 % by weight, based on the total weight of the composition. The weight ratio microcrystalline wax : paraffinum liquidum preferably is from 3:1 to 1:3, e.g., from 2.5:1 to 1.5:1.

In this regard, it is noted that mixtures of microcrystalline wax and paraffinum liquidum are sometimes also referred to as "microcrystalline wax", "cera microcristallina" or "vaseline" (now a registered trade name of CheseBorough Ponds). However, as used herein and in the appended claims the term "microcrystalline wax" denotes exclusively the solid lipid (wax) without paraffinum liquidum.

While the lipids contained in the composition of the present invention may include substances of diverse structures and types, silicone oils and silicone waxes are preferably not present in the composition of the present invention. If they are present, their concentration will usually not be higher than 2 % by weight, e.g., not higher than 0.1 % by weight, or not higher than 0.1 % by weight.

The composition of the present invention also comprises one or more non-ionic emulsifiers (and is preferably free of anionic, cationic and zwitterionic emulsifiers). Preferably, the non-ionic emulsifiers comprise at least two, more preferably at least three different kinds of non-ionic emulsifiers (preferably including oil-in-water emulsifiers) selected from fatty acids, fatty alcohols and alkoxylated (preferably ethoxylated and/or propoxylated) fatty alcohols.

Preferred examples of fatty acids for use in the present invention include saturated or unsaturated fatty acids having from 14 to 22 carbon atoms such as, e.g., palmitic acid, stearic acid, myristic acid, arachidic acid, oleic acid, etc. Preferably, at least two different fatty acids will be present. For example, mixtures of at least three, e.g., at least four, or at least five different fatty acids may be present. The one or more fatty acids will usually be present in a total concentration of at least 1 % by weight, e.g., at least 2 % by weight, or at least 3 % by weight, based on the total weight of the composition, but will usually be present in a concentration of not higher than 6 % by weight, e.g., not higher than 5 % by weight, or not higher than 4.5 % by weight.

Preferred examples of fatty alcohols for use in the present invention include saturated or unsaturated fatty alcohols having from 14 to 22 carbon atoms such as, e.g., palmityl alcohol, myristyl alcohol, oleyl alcohol, stearyl alcohol and cetyl alcohol (optionally present in the form of cetearyl alcohol). Preferably, at least two different fatty alcohols will be present. Further, at least cetyl alcohol or stearyl alcohol and even more preferably, both cetyl alcohol and stearyl alcohol will be present (preferably in a weight ratio of from 1.25:1 to 1:1.25) in the composition of the present invention. The one or more fatty alcohols will usually be present in a total concentration of at least 4 % by weight, e.g., at least 5 % by weight, or at least 6 % by weight, based on the total weight of the composition, but will usually be present in a concentration of not higher than 20 % by weight, e.g., not higher than 17 % by weight, or not higher than 16 % by weight.

Preferred examples of alkoxylated (e.g.,ethoxylated and/or propoxylated) fatty alcohols for use in the present invention include saturated or unsaturated fatty alcohols having from 14 to 22 carbon atoms such as, e.g., palmityl alcohol, myristyl alcohol, oleyl alcohol, stearyl alcohol and cetyl alcohol, which fatty alcohols are present in ethoxylated and/or propoxylated form. Usually, the (total average) number of ethoxy and/or propoxy units per fatty alcohol molecule will be from 2 to 40, e.g., 2, 3, 4, 5, 6, 7, 8, 9, 10, 11, 12, 13, 14, 15, 16, 17, 18, 19, 20, 21, 22, 23, 24, 25, 26, 27, 28, 29, 30 or 35 alkoxy (e.g., ethoxy and/or propoxy) units per fatty alcohol molecule. Preferably, the composition comprises at least two different ethoxylated and/or propoxylated fatty alcohols, e.g., a first fatty alcohol in ethoxylated form and a second fatty alcohol, different from the first fatty alcohol, in propoxylated form. A non-limiting and preferred example of a corresponding combination is oleth-20 and PPG-15 stearyl ether (preferably present in a weight ratio of from 1:5 to 1:15). Of course, further ethoxylated and/or propoxylated fatty alcohols may be present such as, e.g., steareth-2 and/or steareth-21. The one or more ethoxylated and/or propoxylated fatty alcohols will usually be present in a total concentration of at least 7 % by weight, e.g., at least 8 % by weight, or at least 9 % by weight, based on the total weight of the composition, but will usually be present in a concentration of not higher than 20 % by weight, e.g., not higher than 18 % by weight, or not higher than 15 % by weight.

The one or more inorganic substances which are capable of solidifying the emulsion (preferably by binding water, e.g., by absorbing and/or adsorbing water without dissolving in water) may be selected from a wide variety of substances. The component (iv) comprises one or more inorganic aluminum and/or zirconium compounds. Preferred non-limiting examples of corresponding compounds include aluminum chloride, aluminum chlorohydrate, aluminum sesquichlorohydrate, aluminum zirconium trichlorohydrate glycine, aluminum zirconium tetrachlorohydrate glycine, aluminum zirconium pentachlorohydrate glycine, potassium aluminum sulfate (alum). Aluminum chloride and aluminum chlorohydrate are particularly preferred for the purposes of the present invention.

Since the composition of the present invention is a (solid) emulsion, preferably an oil-in-water emulsion, it contains water. Usually the concentration of water in the composition will be at least 30 % by weight, e.g., at least 35 % by weight, at least 40 % by weight, or at least 45 % by weight, based on the total weight of the composition, but usually not higher than 70 % by weight, e.g., not higher than 60 % by weight.

The composition of the present invention may further contain one or more additional substances which are conventionally used in cosmetic compositions, at least as long as they do not prevent the formation of an emulsion and the solidification of the composition and do not adversely affect the desired properties of the composition.

Non-limiting examples of corresponding substances include dyes and coloring pigments, moisturizing and/or humectant substances (such as, e.g., glycerol, urea, and certain amino acids), fillers (such as, e.g., aluminum starch octenylsuccinate), thickeners (e.g., natural or synthetic polymers such as celluloses, modified celluloses, polyacrylates), UV filter substances, electrolytes (e.g., sea salt), fragrance, preservatives and organic solvents.

A preferred additional (and optional) substance for use in the composition of the present invention is talc. If employed, talc will usually be present in a concentration of not more than 15 % by weight, e.g., not more than 10 % by weight, not more than 7 % by weight, or not more than 5 % by weight.

The composition of the present invention may moreover comprise one or more active ingredients which have a positive influence on skin. Active ingredients for use in the present invention preferably exhibit a skin moisturizing effect and/or strengthen the barrier function of skin and/or promote the restructuring of the connective tissue and/or support the function of dry skin and/or positively influence irritated skin (both sensitive skin in general and skin irritated by noxae such as UV light or chemicals) and/or reduce wrinkles and/or protect esthetically unattractive skin such as aged skin and/or improve the appearance of dry or rough skin and/or reduce hyperpigmentation, hypopigmentation, defective pigmentation and/or age spots and/or reduce itching and/or visible blood vessel dilation such as teleangiektasis or cuperosis.

Non-limiting specific examples of active ingredients which may be comprised in the composition of the present invention include bioquinones such as, e.g., ubiquinone Q10, isoflavone and isoflavonoids as well as isoflavonoid containing plant extracts such as soy and clover extracts, flavonoids, genistein, arctiin, cardiolipin, anti-freezing proteins, hop extracts, hop-malt extracts, ascorbic acid and derivatives thereof, tocopherol and esters thereof, biotin, creatine, creatinine, propionic acid, green tea extracts and solutions, white tea extracts and solutions, sericosides, various extracts of licorice root, licochalcone A, silymarin, silyphos, dexpanthenol, ethanol, inhibitors of the prostaglandin metabolism and in particular, cyclooxygenase inhibitors, inhibitors of the leucotriene metabolism and in particular, 5-lipoxygenase inhibitors, inhibitors of the 5-lipoxygenase inhibitor protein, FLAP, folic acid, phytoene, flavone glycosides such as, e.g., α-glucosylrutin, carnitine, polydocanol, carotenoids, taurine, dihydroxyacetone, 8-hexadecene-1,16 dicarboxylic acid, retinol and esters thereof, vitamin E and derivatives thereof, long chain hyaluronic acids (e.g., those having an average molecular weight of from 1 to 3 million Dalton), and short chain hyaluronic acids (e.g., those having an average molecular weight of from 5,000 to 1 million Dalton).

The one or more active ingredients, if present, will usually be present in a total concentration of from 0.1 % to 10 % by weight, based on the total weight of the composition.

The composition of the present invention is preferably free of surfactants and in particular, surfactants which can cause skin irritation. While some of the non-ionic emulsifiers which are or may be present in the present composition may have surfactant properties they are not usually considered to be surfactants. Accordingly, the term "surfactants" as used herein denotes classical surfactants which exhibit cleansing properties and are usually ionic in nature.

The following examples illustrate the present invention without limiting it. The indicated numbers represent percentages by weight, based on the total weight of the composition.

| **Ingredient** | **Ex. 1** | **Ex. 2** | **Ex. 3** | **Ex. 4** | **Ex. 5** | **Ex. 6** | **Ex. 7** |
|---|---|---|---|---|---|---|---|
| Microcrystalline wax + Paraffinum Liquidum (weight ratio 3:1-1:3) | 15.0 | 21.0 | 15.0 | 10.0 | 25.0 | 5.0 | 12.0 |
| Hydrogenated castor oil | 6.0 | 5.0 | 6.0 | 3.0 | 4.0 | 10.0 | 12.0 |
| Palmitic Acid + Stearic Acid + Myristic Acid + Arachidic Acid + Oleic Acid* | 3.8 | 2.4 | 3.8 | 4.7 | 1.1 | 3.5 | 4.0 |
| Cetyl alcohol | 3.0 | 1.0 | 3.0 | 10.0 | 8.0 | 7.0 | 5.0 |
| Stearyl alcohol | 3.5 | 5.0 | 3.5 | 6.2 | 3.1 | 10.0 | 2.0 |
| PPG-15 Stearyl Ether | 10.0 | 15.0 | 10.0 | 3.0 | 13.0 | 5.0 | 12.0 |
| Oleth-20 | 1.0 | 1.5 | 1.0 | 2.0 | 2.5 | 0.5 | 3.0 |
| Steareth-2 | 0.5 | 0.2 | 0.5 | 0.75 | 1.0 | 1.0 | 1.6 |
| Steareth-21 | 0.5 | 1.0 | 0.5 | 1.0 | 0.75 | 0.2 | 1.5 |
| Aluminum chlorohydrate + Water (weight ratio 1:1) | 5.0 | 1.0 | 5.0 | 16.0 | 2.0 | 26.0 | 20.0 |
| Talc | 5.0 | 8.0 | 5.0 | 4.0 | 2.0 | 1.0 | 0.5 |
| Water | 46.7 | 38.9 | 46.7 | 39.35 | 37.55 | 30.8 | 26.4 |

| | | | | | | | |
|---|---|---|---|---|---|---|---|
| *commercially available as Palmac 55-16 from the IOI Oleochemical Group, Omyacid 48 from Omya Peralta, MASCID_1802 from P.T. Musim Mas, Pristerene 4960 from Croda. | | | | | | | |

It is noted that the foregoing examples have been provided merely for the purpose of explanation and are in no way to be construed as limiting of the present invention.

## Claims

1. A cosmetic skin care composition, wherein the composition is an emulsion which is solid up to a temperature of at least 35° C and comprises (i) one or more solid lipids, (ii) one or more liquid lipids, (iii) one or more non-ionic emulsifiers and (iv) one or more inorganic substances which are capable of solidifying the emulsion selected from one or more aluminum and/or zirconium compounds.

2. The composition of claim 1, wherein the composition is solid up to temperature of at least 45° C.

3. The composition of any one of claims 1 and 2, wherein the composition is an oil-in-water emulsion.

4. The composition of any one of claims 1 to 3, wherein (i) comprises a hydrocarbon, in particular a microcrystalline wax, and/or an ester wax, in particular hydrogenated castor oil.

5. The composition of any one of claims 1 to 4, wherein (ii) comprises a hydrocarbon oil, in particular liquid paraffin.

6. The composition of any one of claims 1 to 5, wherein (iii) comprises at least one of a fatty acid, a fatty alcohol, an ethoxylated fatty alcohol and/or a propoxylated fatty alcohol.

7. The composition of claim 6, wherein (iii) comprises one or more C₁₄₋₂₂ fatty acids, in particular one or more of palmitic acid, stearic acid, myristic acid, arachidic acid and/or oleic acid.

8. The composition of claims 6, wherein (iii) comprises one or both of cetyl alcohol and stearyl alcohol.

9. The composition of any one of claims 1 to 8, wherein (iv) comprises one or more aluminum chloride, aluminum chlorohydrate, aluminum sesquichlorohydrate, aluminum zirconium trichlorohydrate glycine, aluminum zirconium tetrachlorohydrate glycine, aluminum zirconium pentachlorohydrate glycine, potassium aluminum sulfate.

10. The composition of any one of claims 1 to 9, wherein the composition comprises at least 30 % by weight of water, from 15 % to 40 % by weight of (i), from 5 % to 20 % by weight of (ii), from 10 % to 30 % by weight of (iii) and/or from 0.5 % to 25 % by weight of (iv), based on a total weight of the composition.

11. A cosmetic shower composition, wherein the composition is an oil-in-water emulsion which is solid up to a temperature of at least 40° C and comprises, based on a total weight of the composition, from 18 % to 25 % by weight of (i) one or more solid lipids, from 7 % to 15 % by weight of (ii) one or more liquid lipids, from 15 % to 25 % by weight of (iii) one or more non-ionic emulsifiers, from 1 % to 15 % by weight of (iv) one or more inorganic aluminum and/or zirconium compounds which are capable of solidifying the emulsion, and at least 35 % by weight of water.

12. The composition of any one of claims 1 to 11, wherein the composition further comprises from 0.5 % to 8 % by weight of talc, based on the total weight of the composition.

13. The composition of any one of claims 1 to 12, wherein the composition is present in the form of solid piece, in particular a bar.

14. A method of skin care while showering, wherein the method comprises cleansing the skin, contacting the cleansed skin with the composition of any one of claims 1 to 13, and thereafter rinsing the skin with water.

15. A method of solidifying a skin care emulsion which comprises one or more solid lipids, one or more liquid lipids, and one or more non-ionic emulsifiers, wherein the method comprises incorporating in the emulsion one or more inorganic substances which are capable of solidifying the emulsion selected from one or more aluminum and/or zirconium compounds.

## Patentansprüche

1. Kosmetische Hautpflegezusammensetzung, wobei es sich bei der Zusammensetzung um eine Emulsion handelt, die bis zu einer Temperatur von mindestens 35 °C fest ist und (i) ein oder mehrere feste Lipide, (ii) ein oder mehrere flüssige Lipide, (iii) einen oder mehrere nichtionische Emulgatoren und (iv) ein oder mehrere anorganische Substanzen, die zur Verfestigung der Emulsion in der Lage sind und aus einer oder mehreren Aluminium- und/oder Zirconiumverbindungen ausgewählt sind, umfasst.

2. Zusammensetzung nach Anspruch 1, wobei die Zusammensetzung bis zu einer Temperatur von mindestens 45 °C fest ist.

3. Zusammensetzung nach einem der Ansprüche 1 und 2, wobei es sich bei der Zusammensetzung um eine Ölin-Wasser-Emulsion handelt.

4. Zusammensetzung nach einem der Ansprüche 1 bis 3, wobei (i) einen Kohlenwasserstoff, insbesondere ein mikrokristallines Wachs, und/oder ein Esterwachs, insbesondere hydriertes Ricinusöl, umfasst.

5. Zusammensetzung nach einem der Ansprüche 1 bis 4, wobei (ii) ein Kohlenwasserstofföl, insbesondere Paraffinöl, umfasst.

6. Zusammensetzung nach einem der Ansprüche 1 bis 5, wobei (iii) eine Fettsäure, einen Fettalkohol, einen ethoxylierten Fettalkohol und/oder einen propoxylierten Fettalkohol umfasst.

7. Zusammensetzung nach Anspruch 6, wobei (iii) ein oder mehrere C₁₄₋₂₂-Fettsäuren, insbesondere Palmitinsäure, Stearinsäure, Myristinsäure, Arachidinsäure und/oder Ölsäure, umfasst.

8. Zusammensetzung nach Anspruch 6, wobei (iii) Cetylalkohol und/oder Stearylalkohol umfasst.

9. Zusammensetzung nach einem der Ansprüche 1 bis 8, wobei (iv) Aluminiumchlorid, Aluminiumchlorhydrat, Aluminiumsesquichlorhydrat, Aluminiumzirconiumtrichlorhydrat-Glycin, Aluminiumzirconiumtetrachlorhydrat-Glycin, Aluminiumzirconiumpentachlorhydrat-Glycin und/oder Kaliumaluminiumsulfat umfasst.

10. Zusammensetzung nach einem der Ansprüche 1 bis 9, wobei die Zusammensetzung mindestens 30 Gew.-% Wasser, 15 bis 40 Gew.-% (i), 5 bis 20 Gew.-% (ii), 10 bis 30 Gew.-% (iii) und/oder 0,5 bis 25 Gew.-% (iv), bezogen auf das Gesamtgewicht der Zusammensetzung, umfasst.

11. Kosmetische Duschzusammensetzung, wobei es sich bei der Zusammensetzung um eine Öl-in-Wasser-Emulsion handelt, die bis zu einer Temperatur von mindestens 40 °C fest ist und, bezogen auf das Gesamtgewicht der Zusammensetzung, 18 bis 25 Gew.-% (i) eines oder mehrerer fester Lipide, 7 bis 15 Gew.-% (ii) eines oder mehrerer flüssiger Lipide, 15 bis 25 Gew.-% (iii) eines oder mehrerer nichtionischer Emulgatoren, 1 bis 15 Gew.-% (iv) einer oder mehrerer anorganischer Aluminium-und/oder Zirconiumverbindungen, die zur Verfestigung der Emulsion in der Lage sind, und mindestens 35 Gew.-% Wasser umfasst.

12. Zusammensetzung nach einem der Ansprüche 1 bis 11, wobei die Zusammensetzung ferner 0,5 bis 8 Gew.-% Talk, bezogen auf das Gesamtgewicht der Zusammensetzung, umfasst.

13. Zusammensetzung nach einem der Ansprüche 1 bis 12, wobei die Zusammensetzung in Form eines festen Stücks, insbesondere eines Riegels, vorliegt.

14. Verfahren zur Hautpflege beim Duschen, wobei das Verfahren das Reinigen der Haut, das Inkontaktbringen der gereinigten Haut mit der Zusammensetzung nach einem der Ansprüche 1 bis 13 und das nachfolgende Abspülen der Haut mit Wasser umfasst.

15. Verfahren zum Verfestigen einer Hautpflegeemulsion, die ein oder mehrere feste Lipide, ein oder mehrere flüssige Lipide und einen oder mehrere nichtionische Emulgatoren umfasst, wobei das Verfahren das Einarbeiten einer oder mehrerer anorganischer Substanzen, die zur Verfestigung der Emulsion in der Lage sind und aus einer oder mehreren Aluminium- und/oder Zirconiumverbindungen ausgewählt sind, in die Emulsion umfasst.

## Revendications

1. Composition cosmétique de soin de la peau, la composition étant une émulsion qui est solide jusqu'à une température d'au moins 35°C et comprenant (i) un ou plusieurs lipides solides, (ii) un ou plusieurs lipides liquides, (iii) un ou plusieurs émulsifiants non ioniques et (iv) une ou plusieurs substances inorganiques qui sont capables de solidifier l'émulsion, choisies parmi un ou plusieurs composés à base d'aluminium et/ou de zirconium.

2. Composition selon la revendication 1, la composition étant solide jusqu'à une température d'au moins 45°C.

3. Composition selon l'une quelconque des revendications 1 et 2, la composition étant une émulsion huile-dans-eau.

4. Composition selon l'une quelconque des revendications 1 à 3, dans laquelle (i) comprend un hydrocarbure, en particulier une cire microcristalline, et/ou une cire d'ester, en particulier de l'huile de ricin hydrogénée.

5. Composition selon l'une quelconque des revendications 1 à 4, dans laquelle (ii) comprend une huile hydrocarbonée, en particulier de la paraffine liquide.

6. Composition selon l'une quelconque des revendications 1 à 5, dans laquelle (iii) comprend au moins l'un parmi un acide gras, un alcool gras, un alcool gras éthoxylé et/ou un alcool gras propoxylé.

7. Composition selon la revendication 6, dans laquelle (iii) comprend un ou plusieurs acides gras en C₁₄₋₂₂, en particulier un ou plusieurs parmi l'acide palmitique, l'acide stéarique, l'acide myristique, l'acide arachidique et/ou l'acide oléique.

8. Composition selon la revendication 6, dans laquelle (iii) comprend l'un, ou les deux, parmi l'alcool cétylique et l'alcool stéarylique.

9. Composition selon l'une quelconque des revendications 1 à 8, dans laquelle (iv) comprend un ou plusieurs parmi le chlorure d'aluminium, le chlorohydrate d'aluminium, le sesquichlorohydrate d'aluminium, le trichlorohydrate d'aluminium et de zirconium avec glycine, le tétrachlorohydrate d'aluminium et de zirconium avec glycine, le pentachlorohydrate d'aluminium et de zirconium avec glycine, le sulfate de potassium et d'aluminium.

10. Composition selon l'une quelconque des revendications 1 à 9, la composition comprenant au moins 30% en poids d'eau, de 15% à 40% en poids de (i), de 5% à 20% en poids de (ii), de 10% à 30% en poids de (iii) et/ou de 0,5% à 25% en poids de (iv), sur la base du poids total de la composition.

11. composition cosmétique pour la douche, la composition étant une émulsion huile-dans-eau qui est solide jusqu'à une température d'au moins 40°C et comprenant, sur la base du poids total de la composition, de 18% à 25% en poids (i) d'un ou plusieurs lipides solides, de 7% à 15% en poids (ii) d'un ou plusieurs lipides liquides, de 15% à 25% en poids (iii) d'un ou plusieurs émulsifiants non ioniques, de 1% à 15% en poids (iv) d'un ou plusieurs composés inorganiques à base d'aluminium et/ou de zirconium qui sont capables de solidifier l'émulsion, et au moins 35% en poids d'eau.

12. Composition selon l'une quelconque des revendications 1 à 11, la composition comprenant en outre de 0,5% à 8% en poids de talc, sur la base du poids total de la composition.

13. Composition selon l'une quelconque des revendications 1 à 12, la composition étant présente sous la forme d'un morceau solide, en particulier d'un pain.

14. Méthode de soin de la peau sous la douche, la méthode comprenant le nettoyage de la peau, la mise en contact de la peau nettoyée avec la composition selon l'une quelconque des revendications 1 à 13, puis le rinçage de la peau par de l'eau.

15. Méthode de solidification d'une émulsion de soin de la peau qui comprend un ou plusieurs lipides solides, un ou plusieurs lipides liquides, et un ou plusieurs émulsifiants non ioniques, la méthode comprenant l'incorporation dans l'émulsion d'une ou plusieurs substances inorganiques qui sont capables de solidifier l'émulsion, choisies parmi un ou plusieurs composés à base d'aluminium et/ou de zirconium.
